# FASCICULE DE BREVET EUROPEEN

(11) **EP 2 803 659 B1**
(45) Date de publication et mention de la délivrance du brevet: **04.01.2017**
(21) Numéro de dépôt: 14168559.4
(22) Date de dépôt: 16.05.2014
(51) Int. Cl.: C07C 253/00, C07D 223/16

(54) **Procédé de synthèse du 3,4-diméthoxybicyclo[4.2.0]octa-1,3,5-triène-7-carbonitrile, et application à la synthèse de l'ivabradine et de ses sels d'addition à un acide pharmaceutiquement acceptable**
Syntheseverfahren von 3,4-(Dimethoxybicyclo[4.2.0]octa-1,3,5-trien-7-carbonitril, und Anwendung zur Synthese von Ivabradin und seinen Additionssalzen mit einer pharmazeutisch akzeptablen Säure
Method for synthesising 3,4-dimethoxybicyclo[4.2.0]octa-1,3,5-triene-7-carbonitrile and use for synthesising ivabradine and the added salts thereof with a pharmaceutically acceptable acid

(30) Priorité: 17.05.2013 FR 1354504
(43) Date de publication de la demande: 19.11.2014
(73) Titulaire: Les Laboratoires Servier, 92284 Suresnes Cedex (FR)
(72) Inventeur: Vaysse-Ludot, Lucile, 76490 Saint-Wandrille-Rancon (FR); Le Flohic, Alexandre, 76640 Fauville en Caux (FR); Vaultier, Michel, 35410 Chateaugiron (FR); Pucheault, Mathieu, 33360 Camblanes et Meynac (FR); Kaminski, Thomas, 35000 Rennes (FR)

(56) Documents cités:
- WO-A1-2011/138625
- TETSUJI KAMETANI ET AL: "Regioselectivity in the thermal 1,2-cycloaddition of benzocyclobutenes to 3,4-dihydroisoquinolines", JOURNAL OF THE CHEMICAL SOCIETY, PERKIN TRANSACTIONS 1, 1 janvier 1974 (1974-01-01), pages 1712-1714, XP055097443, ISSN: 0300-922X, DOI: 10.1039/p19740001712
- HAROLD HART ET AL: "Synthesis of Benzocyclobutenes from Trichloromethylbenzenes 1a", THE JOURNAL OF ORGANIC CHEMISTRY, vol. 31, no. 7, 1 juillet 1966 (1966-07-01), pages 2244-2250, XP055096673, ISSN: 0022-3263, DOI: 10.1021/jo01345a038
- VAN LEUSEN D. ET AL.: "Synthetic uses of tosylmethyl isocyanide (TosMIC)", ORGANIC REACTIONS, vol. 57, 15 avril 2004 (2004-04-15), XP002719004, DOI: 10.1002/0471264180.or057.03
- GOVERDHAN MEHTA ET AL: 'Recent chemistry of benzocyclobutenes' TETRAHEDRON vol. 57, no. 4, 01 Janvier 2001, AMSTERDAM, NL, pages 625 - 659, XP055284353 DOI: 10.1016/S0040-4020(00)00958-3 ISSN: 0040-4020
- ANIL K. SADANA ET AL: 'Cyclobutarenes and Related Compounds' CHEMICAL REVIEWS vol. 103, no. 4, 01 Avril 2003, US, pages 1539 - 1602, XP055284354 DOI: 10.1021/cr010022j ISSN: 0009-2665

## Description

La présente invention concerne un procédé de synthèse du 3,4-diméthoxybicyclo[4.2.0]octa-1,3,5-triène-7-carbonitrile de formule (I) : 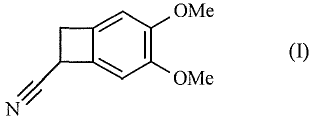 et son application à la synthèse de l'ivabradine et de ses sels d'addition à un acide pharmaceutiquement acceptable.

Le composé de formule (I) obtenu selon le procédé de l'invention est utile dans la synthèse de l'ivabradine de formule (II) : 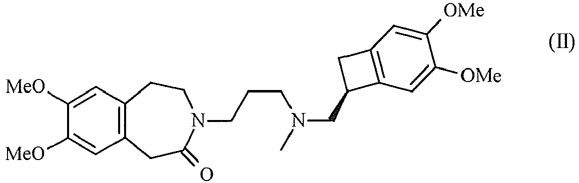 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H-*3-benzazépin-2-one,
de ses sels d'addition à un acide pharmaceutiquement acceptable et de ses hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859.

Ce brevet décrit la préparation de l'ivabradine à partir du 3,4-diméthoxybicyclo[4.2.0]octa-1,3,5-triène-7-carbonitrile de formule (I) : 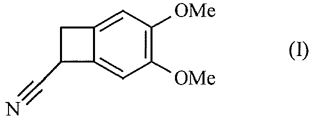 qui est transformé en composé de formule (III) : 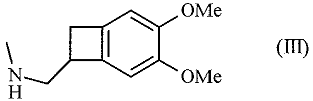 qui est dédoublé pour conduire au composé de formule (IV) : 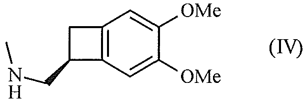 qui est mis en réaction avec le composé de formule (V) : 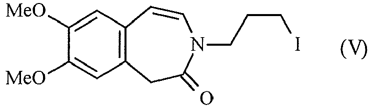 pour conduire au composé de formule (VI) : 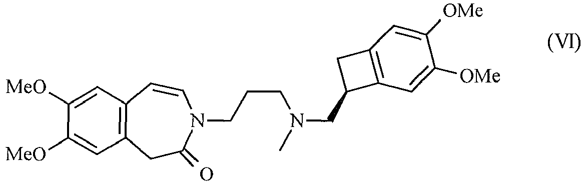 dont l'hydrogénation catalytique conduit à l'ivabradine, qui est alors transformée en son chlorhydrate.

Compte-tenu de l'intérêt industriel de l'ivabradine et de ses sels, il était impératif de trouver un procédé performant permettant d'accéder au composé de formule (I) avec un bon rendement.

La demande WO 2011/138 625 décrit la préparation du composé de formule (I) par cyclisation intramoléculaire du 3-(2-bromo-4,5-diméthoxyphényl)propanenitrile en présence de diéthylamidure de lithium ou de diisopropylamidure de lithium.

La présente invention concerne un procédé de synthèse du composé de formule (I) : 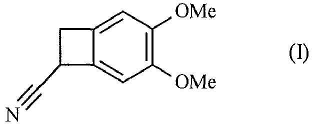 caractérisé en ce que le composé de formule (VII) : 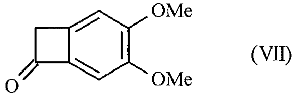 est soumis à l'action du 1-(isocyanométhylsulfonyl)-4-méthylbenzène (TosMIC) en présence d'une base dans un solvant organique ou un mélange de solvants organiques pour conduire au composé de formule (I).

La quantité de 1-(isocyanométhylsulfonyl)-4-méthylbenzène préférentiellement utilisée pour effectuer la transformation du composé de formule (VII) en composé de formule (I) est comprise entre 2 et 5 équivalents.

Parmi les bases pouvant être utilisées pour effectuer la transformation du composé de formule (VII) en composé de formule (I), on peut citer à titre non limitatif les bases organiques de type alcoolates comme le *tert*-butanolate de potassium, le *tert*-butanolate de sodium, l'éthanolate de potassium, l'éthanolate de sodium, le méthanolate de potassium ou le méthanolate de sodium.

La base préférentiellement utilisée pour effectuer la transformation du composé de formule (VII) en composé de formule (I) est le *tert*-butanolate de potassium.

Parmi les solvants organiques pouvant être utilisés pour effectuer la transformation du composé de formule (VII) en composé de formule (I), on peut citer à titre non limitatif les alcools tels que méthanol, éthanol, isopropanol, *tert*-butanol, le tétrahydrofurane, l'éthylène glycol ou le diméthylsufoxyde.

Le solvant organique utilisé pour effectuer la transformation du composé de formule (VII) en composé de formule (I) peut également être constitué par un mélange de deux solvants parmi les solvants organiques précédemment cités.

Le solvant préférentiellement utilisé pour effectuer la transformation du composé de formule (VII) en composé de formule (I) est un mélange de tétrahydrofurane et de méthanol.

La transformation du composé de formule (VII) en composé de formule (I) est conduite à une température préférentiellement comprise entre -20 °C et 50°C.

La présente invention concerne également un procédé de synthèse du composé de formule (I) à partir du composé de formule (VII), caractérisé en ce que ledit composé de formule (VII) est préparé à partir du composé de formule (VIII) : 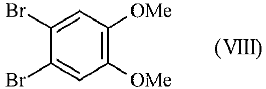 qui est transformé en composé de formule (IX) : 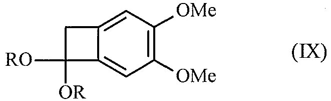 dans laquelle R représente un groupement (C₁-C₄)alkyle,
en présence d'un 1,1-dialkoxyéthène, dont les groupements alkoxyles possèdent de 1 à 4 atomes de carbone, et d'un dérivé organométallique dans un solvant organique,
lequel est transformé en composé de formule (VII) : 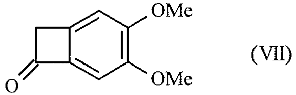 par une réaction d'hydrolyse,
lequel est transformé en produit de formule (I) : 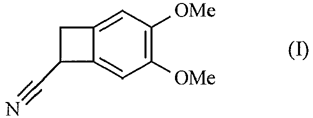 selon le procédé décrit plus haut.

Le 1,1-dialkoxyéthène préférentiellement utilisé pour effectuer la transformation du composé de formule (VIII) en composé de formule (IX) est le 1,1-diéthoxyéthène.

La quantité de 1,1-diéthoxyéthène préférentiellement utilisée pour effectuer la transformation du composé de formule (VIII) en composé de formule (IX) est comprise entre 0,8 et 5 équivalents.

Parmi les dérivés organométalliques pouvant être utilisés pour effectuer la transformation du composé de formule (VIII) en composé de formule (IX), on peut citer à titre non limitatif le *n*-butyllithium, le *s*-butyllithium, le *t*-butyllithium, le phényllithium ou le chlorure d'isopropylmagnésium.

Le dérivé organométallique préférentiellement utilisé pour effectuer la transformation du composé de formule (VIII) en composé de formule (IX) est le *n*-butyllithium.

La quantité de *n*-butyllithium préférentiellement utilisée pour effectuer la transformation du composé de formule (VIII) en composé de formule (IX) est comprise entre 1 et 3 équivalents.

Parmi les solvants organiques pouvant être utilisés pour effectuer la transformation du composé de formule (VIII) en composé de formule (IX), on peut citer à titre non limitatif le toluène, le tétrahydrofurane, le dichlorométhane et le chlorobenzène.

Le solvant préférentiellement utilisé pour effectuer la transformation du composé de formule (VIII) en composé de formule (IX) est le toluène.

La transformation du composé de formule (VIII) en composé de formule (IX) est conduite à une température préférentiellement comprise entre -20 °C et 30°C.

La réaction d'hydrolyse du composé de formule (IX) en composé de formule (VII) peut être effectuée dans un milieu organoaqueux acide composé d'un mélange :
- d'un solvant organique tel que le tétrahydrofurane, l'acétate d'éthyle, le toluène ou le dichlorométhane, et
- d'un acide aqueux tel que l'acide chlorhydrique (1N à 12N) en excès.

Les exemples suivants illustrent l'invention.

Les points de fusion ont été mesurés sur un appareil à point de fusion capillaire de type Buchi Melting point B-545.

Les spectres RMN sont enregistrés sur un appareil Brüker à 400 MHz pour les spectres proton et à 100MHz pour les spectres carbone.
Les déplacements chimiques (δ) sont exprimés en ppm (référence interne TMS).
Les abréviations suivantes ont été utilisées pour qualifier les pics : singulet (s), doublet (d), doublet de doublet (dd), triplet (t), quadruplet (q), multiplet (m).

### Liste des abréviations utilisées

P.F. : point de fusion
THF : tétrahydrofurane
TosMIC : 1-(isocyanométhylsulfonyl)-4-méthylbenzène

### Préparation A : 1,2-dibromo-4,5-diméthoxybenzène

16,16 g de 1,2-diméthoxybenzène (117 mmol) sont agités à 0°C dans du CCl₄ (120 mL). 13,2 mL de dibrome (2,2 éq; 257,4 mmol; 41,13 g) en solution dans CCl₄ (25 mL) sont additionnés goutte-à-goutte (30 min) en surveillant la température (0-5°C) [Placer une sortie qui bulle dans une solution de Na₂CO₃ afin de neutraliser l'acide bromhydrique qui se forme]. Après 2 heures d'agitation à 0 °C, le milieu réactionnel est ensuite versé sur un mélange eau+glace, la phase organique est lavée avec une solution aqueuse de NaHSO₃ à 10% puis avec une solution aqueuse de NaOH à 10%. On obtient après évaporation et séchage 33,42 g d'un solide blanc correspondant au produit du titre.
Rendement = 97%
P.F. 92-93 °C
RMN ¹H (CDCl₃): δ = 7,06 (s; 2H); 3,86 (s; 6H).
RMN ¹³C (CDCl₃): δ = 148,8; 115,9; 114,7; 56,2.

### Préparation B : 1,1-diéthoxyéthène

Le montage se présente comme suit: un ballon de 50 mL muni d'un set de distillation (colonne ∼20 cm; réfrigérant ∼20 cm, thermomètre de distillation). 20 g de 2-bromo-1,1-diéthoxyéthane (101,25 mmol) est additionné rapidement (1 minute) à du *tert*-butanolate de potassium (102 mmol; 11,4 g) refroidi dans un bain de glace. Il se forme une fumée blanche très dense. Lorsque la réaction est terminée (5-10 minutes), le milieu réactionnel est chauffé à 120-130°C (lecture plaque chauffante), et le tertio-butanol généré pendant la réaction est distillé à pression atmosphérique. Lorsque tout le tertio-butanol a été distillé, une trompe à eau est branchée sur le set de distillation. De cette manière, le produit attendu est distillé sous vide en quelques secondes. On obtient 8,5g d'un liquide incolore, contenant des traces de tertio-butanol.
Rendement = 72%
RMN ¹H (CDCl₃): δ = 3,78 (q; 4H); 3,03 (s; 2H); .1,25 (t; 6H).

### Exemple 1 : 7,7-diéthoxy-3,4-diméthoxybicyclo[4.2.0]octa-1,3,5-triène

2,55 g de 1,2-dibromo-4,5-diméthoxybenzène (8,62 mmol; 2 éq), 500 mg de 1,1-diéthoxyéthène (4,31 mmol; 1 éq) dans 25 mL de toluène sont agités à 0°C sous argon. 3,5 mL de *n*-butyllitium (2,5M dans l'hexane, 8,62 mmol; 2 éq) sont additionnés goutte-à-goutte à 0°C. Lorsque l'addition est terminée, le milieu réactionnel est agité 22 heures à température ambiante. Le milieu réactionnel est ensuite hydrolysé et extrait 3 fois à l'acétate d'éthyle. Les phases organiques sont séchées et évaporées, et le brut est purifié par colonne chromatographique sur gel de silice (éluant : heptane/acétate d'éthyle 90/10). On obtient 333 mg d'une huile jaune qui cristallise à température ambiante.
Rendement =31%
RMN ¹H (CDCl₃):δ = 6,86 (s; 1H); 6,79 (s; 1H); 3,84 (s; 6H); 3,72 (q; 4H); 3,30 (s; 2H); .1,25 (t; 6H).

### Exemple 2 : 3,4-diméthoxybicyclo[4.2.0]octa-1,3,5-trién-7-one

Le 7,7-diéthoxy-3,4-diméthoxy-bicyclo[4.2.0]octa-1,3,5-triène (1,76 g, 6,98 mmol) est agité dans un mélange THF/eau (6/1) à température ambiante. 815 mg d'une solution aqueuse d'HCl 11N (1,1eq, 7,7 mmol) sont ensuite ajoutés. Le milieu réactionnel est agité pendant 2h à température ambiante. De l'eau est ajoutée pour faciliter les deux extractions avec l'acétate d'éthyle (2x30 mL). Les phases organiques sont séchées sur MgSO₄, puis mises à sec. On obtient 1,01 g de produit du titre sous forme d'une poudre grise.
Rendement =81%
RMN ¹H (CDCl₃): 7,02 (s; 2H); 6,82 (s; 2H); 3,99 (s; 3H); 3,87 (s; 5H).
RMN¹³C (CDCl₃): 185,8; 155,9; 151,4; 146,2; 138,7; 105,6; 102,3; 56,4; 56,1; 51,0.
P.F. = 146 °C

### Exemple 3 : 3,4-diméthoxybicyclo[4.2.0]octa-1,3,5-triène-7-carbonitrile

Une solution de TosMIC (0,98 g, 4,98 mmol, 2,3 éq) dans le THF (3 mL) est coulée en 20 minutes sur une solution de *tert-butanolate* de potassium (1,22 g ; 10,9 mmol ; 5 éq) dans le THF (7,5 mL) agité à 0°C sous azote. Puis 200 µL de méthanol sont ajouté sur le milieu et l'agitation est maintenue pendant 30 minutes à 0°C. Parallèlement, dans un second tricol, on charge la 3,4-diméthoxybicyclo[4.2.0]octa-1,3,5-trién-7-one (0,39 g, 2,17 mmol, 1 éq), le bromure de lithium (0,19 g, 2,17 mmol, 1 éq) et le THF (2,5 mL). Après refroidissement à 0°C sous azote, la solution de TosMIC et de *tert-butanolate* de potassium est transférée sur le milieu réactionnel. Après retour à température ambiante, la solution est portée à 40°C et agitée pendant 16h à cette température. Le milieu est ensuite hydrolysé avec une solution de HCl 11N (0,7 mL, 7,73 mmol) dans l'eau (2 mL). Après évaporation sous pression réduite du THF, le produit est extrait par 5 mL de dichlorométhane. La phase organique est lavée deux fois par 2 x 5 mL d'eau avant mise à sec. Le produit brut est purifié par colonne chromatographique sur gel de silice avec le mélange binaire méthylcyclohexane/acétate d'éthyle 75/25 pour obtenir le produit du titre sous forme d'une poudre crème.
Rendement = 54%
RMN ¹H (CDCl₃): 6,76 (s; 1H); 6,68 (s; 1H); 4,14 (m; 1H); 3,83 (s; 6H); 3,59-3,41 (m; 2H).
RMN¹³C (CDCl₃): 151,4; 150,4; 134,2; 129,7; 119,9; 106,9; 106,1; 56,2; 35,5; 22,6.

### Exemple 4 : 3,4-diméthoxy-N-méthylbicyclo[4.2.0]octa-1,3,5-trién-7-amine

D'après EP 0 534 859

### Stade 1 : chlorhydrate de 3,4-diméthoxybicyclo[4.2.0]octa-1,3,5-trién-7-amine

On ajoute goutte à goutte et sous agitation à température ambiante, 312 mL d'une solution molaire de borane complexé avec du THF à une solution de 25 g de 3,4-diméthoxybicyclo[4.2.0]octa-1,3,5-triène-7-carbonitrile dans 250 mL de THF. On laisse en contact pendant 12 heures, puis on ajoute 200 mL d'éthanol et on agite 1 heure. On ajoute, goutte à goutte, 100 mL d'éther chlorhydrique 3,3N. On obtient 27,7 g du produit attendu.
Rendement = 90%
P.F. = 205°C

### Stade 2 : (3,4-diméthoxybicyclo[4.2.0]octa-1,3,5-trién-7-yl)carbamate d'éthyle

On coule 1,5 mL de chloroformiate d'éthyle sur une suspension de 3,4 g du composé obtenu au stade 1 de 4,5 mL de triéthylamine et de 50 mL de dichlorométhane. On laisse une nuit sous agitation à température ambiante puis on lave à l'eau et à l'acide chlorhydrique 1N. On sèche et on évapore à sec le solvant. On obtient 3,2 g d'une huile correspondant au produit attendu.
Rendement = 80%

### Stade 3 : 3,4-diméthoxy-N-méthylbicyclo[4.2.0]octa-1,3,5-trién-7-amine

On ajoute 3,2 g du composé obtenu au stade 2 en solution dans 30 mL de THF à une suspension de 0,9 g de LiAlH₄ dans 20 mL de THF. On porte au reflux 1 heure 30 minutes puis on hydrolyse par 0,6 ml d'eau et 0,5 mL de soude à 20%, et enfin par 2,3 mL d'eau. Les sels minéraux sont ensuite filtrés, rincés au THF puis le filtrat obtenu est évaporé à sec. On obtient 2,3 g du composé attendu.
Rendement = 92%

### Exemple 5 : (7S)-3,4-diméthoxy-N-méthylbicyclo[4.2.0]octa-1,3,5-trién-7-amine

D'après EP 0 534 859

On fait réagir l'amine obtenue à l'exemple 4 avec une quantité équimolaire de l'acide (d) camphosulfonique dans l'éthanol. Après évaporation sous vide du solvant, le sel est recristallisé une première fois dans l'acétate d'éthyle puis dans l'acétonitrile jusqu'à l'obtention de l'énantiomère cible avec une pureté optique supérieure à 99% (évaluation par HPLC sur colonne Chiralcel ^{®} OD).

### Exemple 6 : 3-{3-[{[(7S)-3,4-diméthoxybicyclo[4.2.0]octa-1,3,5-trién-7-yl]méthyl}(méthyl)amino]propyl}-7,8-diméthoxy-1,3-dihydro-2H-3-benzazépin-2-one

D'après EP 0 534 859

La solution de sel de (d) camphosulfonate obtenu à l'exemple 5 dans l'acétate d'éthyle est amenée à pH basique à l'aide d'hydroxyde de sodium puis la phase organique est séparée, lavée, séchée sue Na₂SO₄ et évaporée.

On porte ensuite au reflux pendant 18 heures un mélange composé de 5,6 g de carbonate de potassium, 2,2 g de l'amine ci-dessus dans 100 mL d'acétone et de 4g de 3-(3-iodopropyl)-7,8-diméthoxy-1,3-dihydro-2*H*-3-benzazépin-2-one.

### On évapore le solvant sous vide, on reprend le résidu à l'acétate d'éthyle, puis on extrait à l'acide chlorhydrique 3N.

On amène la phase aqueuse décantée à pH basique à l'aide d'hydroxyde de sodium, puis on l'extrait à l'acétate d'éthyle. Après lavage à neutralité et séchage sur MgSO₄, on évapore sous vide pour obtenir 4,5 g d'une huile qui est purifiée sur colonne de silice en utilisant comme éluant un mélange dichlorométhane/méthanol (90/10).
Rendement = 64%

### Exemple 7 : 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

D'après EP 0 534 859

5 g du composé obtenu à l'exemple 6 dans 50 mL d'acide acétique glacial sont hydrogénés dans un appareil de Parr, sous un pression de 4,9 bar d'hydrogène à température ambiante pendant 24 heures, en présence d'1 g d'hydroxyde de palladium à 10%. On filtre le catalyseur, on évapore le solvant, puis on reprend le résidu sec à l'eau et à l'acétate d'éthyle. On sèche la phase organique sur sulfate de magnésium anhydre, on concentre sous vide puis le résidu est purifié sur colonne de silice en utilisant comme éluant un mélange dichlorométhane/méthanol (95/5). Après recristallisation dans l'acétate d'éthyle, on obtient 2g du composé attendu.
Rendement = 40%
P.F. = 101-103°C

## Revendications

1. Procédé de synthèse du composé de formule (I) : 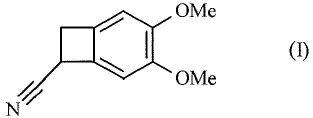 **caractérisé en ce que** le composé de formule (VII) : 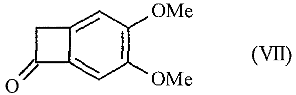 est soumis à l'action du 1-(isocyanométhylsulfonyl)-4-méthylbenzène (TosMIC) en présence d'une base dans un solvant organique pour conduire au composé de formule (I).

2. Procédé selon la revendication 1, **caractérisé en ce que** la quantité de 1-(isocyanométhylsulfonyl)-4-méthylbenzène utilisée pour effectuer la transformation du composé de formule (VII) en composé de formule (I) est comprise entre 2 et 5 équivalents.

3. Procédé selon l'une quelconque des revendications 1 ou 2, **caractérisé en ce que** la base utilisée pour effectuer la transformation du composé de formule (VII) en composé de formule (I) est choisi parmi le *tert*-butanolate de potassium, le *tert-*butanolate de sodium, l'éthanolate de potassium, l'éthanolate de sodium, le méthanolate de potassium ou le méthanolate de sodium.

4. Procédé selon la revendication 3, **caractérisé en ce que** la base utilisée pour effectuer la transformation du composé de formule (VII) en composé de formule (I) est le *tert-*butanolate de potassium.

5. Procédé selon l'une quelconque des revendications 1 à 4, **caractérisé en ce que** le solvant organique utilisé pour effectuer la transformation du composé de formule (VII) en composé de formule (I) est choisi parmi le méthanol, l'éthanol, l'isopropanol, le *tert*-butanol, le tétrahydrofurane, l'éthylène glycol et le diméthylsufoxyde ou un mélange de deux parmi ces solvants.

6. Procédé selon la revendication 5, **caractérisé en ce que** le solvant organique utilisé pour effectuer la transformation du composé de formule (VII) en composé de formule (I) est un mélange de tétrahydrofurane et de méthanol.

7. Procédé selon l'une quelconque des revendications 1 à 6, **caractérisé en ce que** la transformation du composé de formule (VII) en composé de formule (I) est conduite à une température comprise entre -20 °C et 50°C.

8. Procédé selon la revendication 1, **caractérisé en ce que** le composé de formule (VII) est préparé à partir du composé de formule (VIII) : 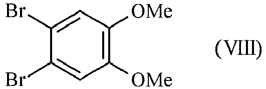 qui est transformé en composé de formule (IX) : 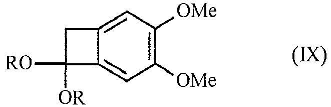 dans laquelle R représente un groupement (C₁-C₄)alkyle,
en présence d'un 1,1-dialkoxyéthène, dont les groupements alkoxyles possèdent de 1 à 4 atomes de carbone, et d'un dérivé organométallique dans un solvant organique,
lequel est hydrolysé dans un milieu organoaqueux acide en composé de formule (VII) :

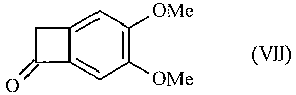

9. Procédé selon la revendication 8, **caractérisé en ce que** le 1,1-dialkoxyéthène utilisé pour effectuer la transformation du composé de formule (VIII) en composé de formule (IX) est le 1,1-diéthoxyéthène.

10. Procédé selon la revendication 9, **caractérisé en ce que** la quantité de 1,1 diéthoxyéthène utilisée pour effectuer la transformation du composé de formule (VIII) en composé de formule (IX) est comprise entre 0,8 et 5 équivalents.

11. Procédé selon l'une quelconque des revendications 8 à 10, **caractérisé en ce que** le dérivé organométallique utilisé pour effectuer la transformation du composé de formule (VIII) en composé de formule (IX) est choisi parmi le *n*-butyllithium, le *s-*butyllithium, le *t*-butyllithium, le phényllithium et le chlorure d'isopropylmagnésium.

12. Procédé selon la revendication 11, **caractérisé en ce que** le dérivé organométallique utilisé pour effectuer la transformation du composé de formule (VIII) en composé de formule (IX) est le *n*-butyllithium.

13. Procédé selon la revendication 12, **caractérisée en ce que** la quantité de *n*-butyllithium utilisée pour effectuer la transformation du composé de formule (VIII) en composé de formule (IX) est comprise entre 1 et 3 équivalents.

14. Procédé selon l'une quelconque des revendications 8 à 13, **caractérisé en ce que** le solvant organique utilisé pour effectuer la transformation du composé de formule (VIII) en composé de formule (IX) est choisi parmi le toluène, le tétrahydrofurane, le dichlorométhane et le chlorobenzène.

15. Procédé selon la revendication 14, **caractérisé en ce que** le solvant organique utilisé pour effectuer la transformation du composé de formule (VIII) en composé de formule (I) est le toluène.

16. Procédé selon l'une quelconque des revendications 1 à 15, **caractérisé en ce que** la transformation du composé de formule (VIII) en composé de formule (IX) est conduite à une température comprise entre -20 °C et 30°C.

17. Procédé de synthèse de l'ivabradine, de ses sels pharmaceutiquement acceptables et de ses hydrates, dans lequel le composé de formule (VII) est transformé en intermédiaire de formule (I) suivant le procédé de la revendication 1, puis l'intermédiaire de formule (I) est transformé en ivabradine.

## Patentansprüche

1. Verfahren zur Synthese der Verbindung der Formel (I): 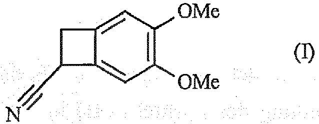 **dadurch gekennzeichnet, dass** die Verbindung der Formel (VII): 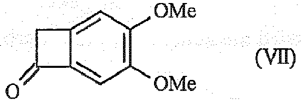 in Gegenwart einer Base in einem organischen Lösungsmittel der Einwirkung von 1-(Isocyanomethylsulfonyl)-4-methylbenzol (TosMIC) unterworfen wird zur Bildung der Verbindung der Formel (I).

2. Verfahren nach Anspruch 1, **dadurch gekennzeichnet, dass** die für die Durchführung der Umwandlung der Verbindung der Formel (VII) in die Verbindung der Formel (I) verwendete Menge von 1-(Isocyanomethylsulfonyl)-4-methylbenzol zwischen 2 und 5 Äquivalenten liegt.

3. Verfahren nach einem der Ansprüche 1 oder 2, **dadurch gekennzeichnet, dass** die zur Durchführung der Umwandlung der Verbindung der Formel (VII) in die Verbindung der Formel (I) verwendete Base ausgewählt wird aus Kalium-*tert*.-butanolat, Natrium-*tert*.-butanolat, Kaliumethanolat, Natriumethanolat, Kaliummethanolat oder Natriummethanolat.

4. Verfahren nach Anspruch 3, **dadurch gekennzeichnet, dass** die zur Durchführung der Umwandlung der Verbindung der Formel (VII) in die Verbindung der Formel (I) verwendete Base Kalium-*tert*.-butanolat ist.

5. Verfahren nach einem der Ansprüche 1 bis 4, **dadurch gekennzeichnet, dass** das zur Durchführung der Umwandlung der Verbindung der Formel (VII) in die Verbindung der Formel (I) verwendete organische Lösungsmittel aus Methanol, Ethanol, Isopropanol, *tert*.-Butanol, Tetrahydrofuran, Ethylenglykol und Dimethylsulfoxid oder einer Mischung von zwei dieser Lösungsmittel ausgewählt wird.

6. Verfahren nach Anspruch 5, **dadurch gekennzeichnet, dass** das zur Durchführung der Umwandlung der Verbindung der Formel (VII) in die Verbindung der Formel (I) verwendete organische Lösungsmittel eine Mischung aus Tetrahydrofuran und Methanol ist.

7. Verfahren nach einem der Ansprüche 1 bis 6, **dadurch gekennzeichnet, dass** die Umwandlung der Verbindung der Formel (VII) in die Verbindung der Formel (I) bei einer Temperatur zwischen -20 °C und 50 °C durchgeführt wird.

8. Verfahren nach Anspruch 1, **dadurch gekennzeichnet, dass** die Verbindung der Formel (VII) hergestellt wird ausgehend von der Verbindung der Formel (VIII): 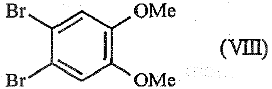 welche in Gegenwart eines 1,1-Dialkoxyethens, dessen Alkoxygruppen 1 bis 4 Kohlenstoffatome aufweisen, und eines metallorganischen Derivats in einem organischen Lösungsmittel in die Verbindung der Formel (IX): 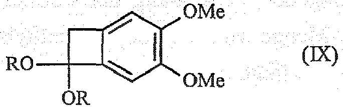 worin R eine (C₁-C₄)-Alkylgruppe bedeutet, umgewandelt wird,
welche in einem sauren wässrig-organischen Medium zu der Verbindung der Formel (VII) hydrolysiert wird:

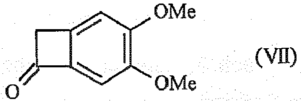

9. Verfahren nach Anspruch 8, **dadurch gekennzeichnet, dass** das zur Durchführung der Umwandlung der Verbindung der Formel (VIII) in die Verbindung der Formel (IX) verwendete 1,1-Dialkoxyethen 1,1-Diethoxyethen ist.

10. Verfahren nach Anspruch 9, **dadurch gekennzeichnet, dass** die zur Durchführung der Umwandlung der Verbindung der Formel (VIII) in die Verbindung der Formel (IX) verwendete Menge von 1,1-Diethoxyethen zwischen 0,8 und 5 Äquivalenten liegt.

11. Verfahren nach einem der Ansprüche 8 bis 10, **dadurch gekennzeichnet, dass** das zur Durchführung der Umwandlung der Verbindung der Formel (VIII) in die Verbindung der Formel (IX) verwendete metallorganische Derivat aus *n*-Butyllithium, *s-*Butyllithium, *tert*.-Butyllithimn, Phenyllithium und Isopropylmagnesiumchlorid ausgewählt wird.

12. Verfahren nach Anspruch 11, **dadurch gekennzeichnet, dass** das zur Durchführung der Umwandlung der Verbindung der Formel (VIII) in die Verbindung der Formel (IX) verwendete metallorganische Derivat *n*-Butyllithium ist.

13. Verfahren nach Anspruch 12, **dadurch gekennzeichnet, dass** die zur Durchführung der Umwandlung der Verbindung der Formel (VIII) in die Verbindung der Formel (IX) verwendete Menge *n*-Butyllithium zwischen 1 und 3 Äquivalenten liegt.

14. Verfahren nach einem der Ansprüche 8 bis 13, **dadurch gekennzeichnet, dass** das zur Durchführung der Umwandlung der Verbindung der Formel (VIII) in die Verbindung der Formel (IX) verwendete organische Lösungsmittel aus Toluol, Tetrahydrofuran, Dichlormethan und Chlorbenzol ausgewählt wird.

15. Verfahren nach Anspruch 14, **dadurch gekennzeichnet, dass** das zur Durchführung der Umwandlung der Verbindung der Formel (VIII) in die Verbindung der Formel (IX) verwendete Lösungsmittel Toluol ist.

16. Verfahren nach einem der Ansprüche 1 bis 15, **dadurch gekennzeichnet, dass** die Umwandlung der Verbindung der Formel (VIII) in die Verbindung der Formel (IX) bei einer Temperatur zwischen -20 °C und 30 °C durchgeführt wird.

17. Verfahren zur Synthese von Ivabradin, seinen pharmazeutisch annehmbaren Salzen und seinen Hydraten, worin die Verbindung der Formel (VII) nach dem Verfahren des Anspruchs 1 in das Zwischenprodukt der Formel (I) umgewandelt wird, wonach das Zwischenprodukt der Formel (I) in Ivabradin umgewandelt wird.

## Claims

1. Process for the synthesis of the compound of formula (I): 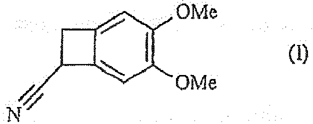 **characterised in that** the compound of formula (VII): 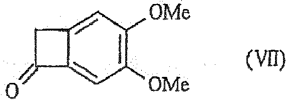 is subjected to the action of 1-(isocyanomethylsulphonyl)-4-methylbenzene (TosMIC) in the presence of a base in an organic solvent to yield the compound of formula (I).

2. Process according to claim 1, **characterised in that** the amount of 1-(isocyano-methylsulphonyl)-4-methylbenzene used to carry out the conversion of the compound of formula (VII) to form the compound of formula (I) is from 2 to 5 equivalents.

3. Process according to either claim 1 or claim 2, **characterised in that** the base used to carry out the conversion of the compound of formula (VII) to form the compound of formula (I) is selected from potassium *tert*-butoxide, sodium *tert-*butoxide, potassium ethoxide, sodium ethoxide, potassium methoxide and sodium methoxide.

4. Process according to claim 3, **characterised in that** the base used to carry out the conversion of the compound of formula (VII) to form the compound of formula (I) is potassium *tert*-butoxide.

5. Process according to any one of claims 1 to 4, **characterised in that** the organic solvent used to carry out the conversion of the compound of formula (VII) to form the compound of formula (I) is selected from methanol, ethanol, isopropanol, *tert*-butanol, tetrahydrofuran, ethylene glycol and dimethyl sulphoxide, or a mixture of two from among those solvents.

6. Process according to claim 5, **characterised in that** the organic solvent used to carry out the conversion of the compound of formula (VII) to form the compound of formula (I) is a mixture of tetrahydrofuran and methanol.

7. Process according to any one of claims 1 to 6, **characterised in that** the conversion of the compound of formula (VII) to form the compound of formula (I) is carried out at a temperature from -20°C to 50°C.

8. Process according to claim 1, **characterised in that** the compound of formula (VII) is prepared starting from the compound of formula (VIII): 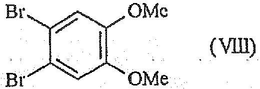 which is converted into a compound of formula (IX): 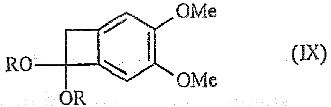 wherein R represents a (C₁-C₄)alkyl group,
in the presence of a 1,1-dialkoxyethene, the alkoxy groups of which have from 1 to 4 carbon atoms, and an organometallic compound in an organic solvent,
which compound of formula (IX) is hydrolysed in an organoaqueous acidic medium to form the compound of formula (VII):

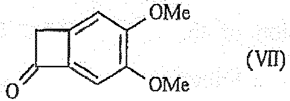

9. Process according to claim 8, **characterised in that** the 1,1-dialkoxyethene used to carry out the conversion of the compound of formula (VIII) to form the compound of formula (IX) is 1,1-diethoxyethene.

10. Process according to claim 9, **characterised in that** the amount of 1,1-diethoxyethene used to carry out the conversion of the compound of formula (VIII) to form the compound of formula (IX) is from 0.8 to 5 equivalents.

11. Process according to any one of claims 8 to 10, **characterised in that** the organometallic compound used to carry out the conversion of the compound of formula (VIII) to form the compound of formula (IX) is selected from *n-*butyllithium, *s*-butyllithium, *t*-butyllithium, phenyllithium and isopropylmagnesium chloride.

12. Process according to claim 11, **characterised in that** the organometallic compound used to carry out the conversion of the compound of formula (VIII) to form the compound of formula (IX) is *n*-butyllithium.

13. Process according to claim 12, **characterised in that** the amount of *n*-butyllithium used to carry out the conversion of the compound of formula (VIII) to form the compound of formula (IX) is from 1 to 3 equivalents.

14. Process according to any one of claims 8 to 13, **characterised in that** the organic solvent used to carry out the conversion of the compound of formula (VIII) to form the compound of formula (IX) is selected from toluene, tetrahydrofuran, dichloromethane and chlorobenzene.

15. Process according to claim 14, **characterised in that** the organic solvent used to carry out the conversion of the compound of formula (VIII) to form the compound of formula (I) is toluene.

16. Process according to any one of claims 1 to 15, **characterised in that** the conversion of the compound of formula (VIII) to form the compound of formula (IX) is carried out at a temperature from -20°C to 30°C.

17. Process for the synthesis of ivabradine, of pharmaceutically acceptable salts thereof and of hydrates thereof, wherein the compound of formula (VII) is converted into the intermediate of formula (I) according to the process of claim 1, and then the intermediate of formula (I) is converted into ivabradine.
